Europäisches Patentamt

European Patent Office

Office européen des brevets

(19)

(11) **EP 0 754 299 B1**

(12) **EUROPÄISCHE PATENTSCHRIFT**

(45) Veröffentlichungstag und Bekanntmachung des Hinweises auf die Patenterteilung:
**25.11.1998 Patentblatt 1998/48**

(21) Anmeldenummer: **95915051.7**

(22) Anmeldetag: **04.04.1995**

(51) Int Cl.⁶: **G01N 33/18**

(86) Internationale Anmeldenummer:
**PCT/AT95/00066**

(87) Internationale Veröffentlichungsnummer:
**WO 95/27200 (12.10.1995 Gazette 1995/43)**

(54) **EINRICHTUNG ZUR BESTIMMUNG VON ABSCHEIDERATEN**

DEVICE FOR DETERMINING DEPOSITION RATES

DISPOSITIF PERMETTANT DE DETERMINER DES VITESSES DE DEPOT

(84) Benannte Vertragsstaaten:
**AT BE CH DE DK ES FR GB IT LI NL**

(30) Priorität: **05.04.1994 AT 699/94**

(43) Veröffentlichungstag der Anmeldung:
**22.01.1997 Patentblatt 1997/04**

(73) Patentinhaber: **MAITRON CHEMIEFREIE WASSERBEHANDLUNG G.M.B.H.**
**6020 Innsbruck (AT)**

(72) Erfinder:
- **LEITER, Klaus**
  **A-6176 Völs (AT)**
- **WALDER, Gerhard**
  **A-6020 Innsbruck (AT)**

(74) Vertreter: **Torggler, Paul, Dr. et al**
**Wilhelm-Greil-Strasse 16**
**6020 Innsbruck (AT)**

(56) Entgegenhaltungen:
| | |
|---|---|
| WO-A-93/08468 | DE-A- 3 933 798 |
| GB-A- 1 564 757 | US-E- R E33 346 |

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft eine Einrichtung zur Bestimmung von Abscheideraten von gelösten Inhaltsstoffen einer Flüssigkeit oder Schmelze an einer Oberfläche

- mit einer beheizten Röhre oder einem Schlauch, durch welche(n) die Flüssigkeit oder Schmelze strömt,
- mit einer Einrichtung zur Erfassung der Durchflußrate durch die Röhre bzw. Schlauch,
- mit einer Einrichtung zur Erfassung der Druckdifferenz zwischen Eingang und Ausgang der Röhre bzw. des Schlauches,
- mit einer Einrichtung zur Erfassung der Außentemperatur der Röhre bzw. des Schlauches.

Die entsprechende Einrichtung gemäß DE-A-3 933 798 erfaßt jedoch nicht die oben genannte Durchdifferenz und Außentemperatur.

Weiters betrifft die Erfindung ein Verfahren zur quantitativen Bestimmung von Abscheideraten von gelösten Inhaltsstoffen einer Flüssigkeit oder Schmelze an einer Oberfläche.

Es sind bereits Einrichtungen bekannt, die in einem miniaturisierten Wärmetauscher Wasser durch eine in einem Wasserbad beheizte Kapillare strömen lassen, um das Bilden von Kalkablagerungen in der Kapillare in relativ kurzer Zeit studieren zu können. Die bekannte Einrichtung nach DE-A-3 933 798 wird so betrieben, daß durch die Röhre bzw. Kapillare Wasser mit einer konstanten Durchflußrate (konstantes Volumen pro Zeiteinheit) gepumpt wird und gleichzeitig der Druck am Eingang der Röhre gemessen wird. Damit läßt sich aus dem Druckanstieg auf eine Verkalkung im Inneren der Kapillare schließen.

Auch aus GB-A-1 564 757 und US-E-RE 33346 sind ähliche Einrichtungen bekannt.

Aufgabe der Erfindung ist es, eine Einrichtung der eingangs genannten Gattung zu schaffen, mit der sich die Abscheiderate von gelösten Inhaltsstoffen einer Flüssigkeit oder Schmelze an einer Oberfläche quantitativ gut und rasch bestimmen lassen.

Erfindungsgemäß wird dies dadurch erreicht, daß neben der bekannten Durchflußraten- und Druckdifferenzmessung eine Einrichtung zur Erfassung der Temperatur der Flüssigkeit bzw. der Schmelze sowohl vor als auch nach der Röhre bzw. dem Schlauch vorgesehen ist.

Erst durch Kenntnis all dieser Meßgrößen ist es moglich, die Abscheiderate als zeitliche Verringerung des Innendurchmessers der Röhre bzw. des Schlauches quantitativ genau zu erfassen. Die Aufwachsrate A ergibt sich somit zu

$$A = \frac{dR}{dt}. \tag{1}$$

Den momentanen Radius kann man nach der Formel von HagenPoiseuille errechnen.

$$Q = \frac{\pi R^4}{8\eta(T)L} \Delta p \tag{2}$$

In dieser Formel bedeutet Q die Durchflußrate, $\Delta p$ die Druckdifferenz zwischen Eingang und Ausgang der Röhre, L die Länge der Röhre und $\eta(T)$ die temperaturabhangige Viskosität der Flüssigkeit bzw. der Schmelze.

Während die Große L bekannt ist und Q und $\Delta p$ leicht meßbar sind, hat man die Problematik, daß die Viskosität von der Temperatur abhängt und man die Temperatur der Flüssigkeit im Inneren der Röhre aufgrund des geringen Durchmessers derselben praktisch nicht messen kann. Aus diesem Grund schlägt die Erfindung im wesentlichen vor, die Temperatur vor und nach der Röhre zu messen. Dies erlaubt es, bei Kenntnis weiterer leicht erfaßbarer bzw. bekannter Parameter auf die mittlere Temperatur innerhalb der Rohre bzw. des Schlauches zu schließen und damit die temperaturabhangige Viskosität zu kennen. Wenn man die temperaturabhängige Viskosität kennt, so kann man aus der Formel 2 bei den ebenfalls erfaßten bzw. bekannten übrigen Parametern leicht den Innenradius R ermitteln, der sich eben beim Aufwachsen von Inhaltsstoffen, beispielsweise Kalk, auf der inneren Oberfläche der Röhre bzw. des Schlauches verringert, ermitteln. Eine quantitative Erfassung der Abscheiderate ist damit möglich.

Nun zurück zur Ermittlung der mittleren Temperatur aus der Messung der übrigen Temperaturen.

Der Temperaturverlauf der Flüssigkeit in einer Röhre (in einem Schlauch), die (der) in einem Wärmebad/Wärmeblock auf der Außenseite beheizt wird, berechnet sich analog dem Temperaturverlauf in einem Röhrenwärmetauscher in einem Temperaturbad.

Für die Austrittstemperatur gilt:

$$T\_aus = T0 + (T\_ein - T0) \cdot exp\left(-\frac{kA}{W}\right) \qquad (3)$$

mit k dem Wärmedurchgangskoeffizienten, A der Außenfläche des Rohres/Schlauches der Länge L (A = $2\pi r_a L$, $r_a$ = Außenradius des Rohres/Schlauchs), W dem Wasserwert (W = Durchflußrate x spezifische Wärmekapazität).
Für den Temperaturverlauf T(z) gilt (Rohr/Schlauch der Länge L in z-Richtung,):

$$T(z) = T0 + (T\_ein - T0) \cdot exp\left(-\frac{k \cdot 2 \cdot \pi \cdot r_a \cdot z}{W}\right) \qquad (4)$$

Die mittlere Temperatur der Flüssigkeit im Rohr/Schlauch (Länge L) berechnet sich folgendermaßen:

$$T_m = \prec T \succ = \frac{1}{L}\int_0^L T(z)dz = T0 + \frac{T\_ein - T0}{\gamma}[1 - exp(-\gamma \cdot L)] \qquad (5)$$

mit $\gamma = 2\pi k r_a/W$.

Auch für die Berechnung der Abscheiderate läßt sich annehmen, daß der Prozeß des Abscheidens verglichen zur Geschwindigkeit der Meßdatenerfassung klein ist, also quasi statisch verläuft. Für jeden Zeitpunkt t ist aus den augenblicklichen Meßgrößen zunächst aus der Gleichung (3) der k-Wert zu berechnen. Daraufhin erlaubt es die Gleichung (5) die mittlere Temperatur Tm zu ermitteln. Aus einer Tabelle kann man dann leicht die temperaturabhängige Viskosität $\eta(T_m)$ ermitteln. Man hat mit $<\eta> = \eta (<T>) = \eta(T_m)$ eine gute Näherung auf ca. 10 %.

Obiger Wert für die mittlere Viskosität $\eta(T_m)$ trägt der starken Nichtlinearität der Temperaturabhängigkeit der Viskosität nicht ganz Rechnung. Exakt berechnet sich die mittlere Viskosität folgendermaßen:

$$\langle \eta \rangle = \frac{\int_0^L \eta(z)dz}{L} = \frac{\int_{T_{ein}}^{T_{aus}} \eta(z(T)) \cdot z'(T) \cdot dT}{T_{aus} - T_{ein}} \qquad (6)$$

z(T) berechnet sich aus der Umkehrung von (4).

Man hat nun alle Größen, um aus der Formel (2) den über die Formel (1) direkt mit der Abscheiderate A korrelierten Radius R zu ermitteln.

Weitere Vorteile und Einzelheiten der Erfindung werden anhand der nachfolgenden Figurenbeschreibung näher erläutert.

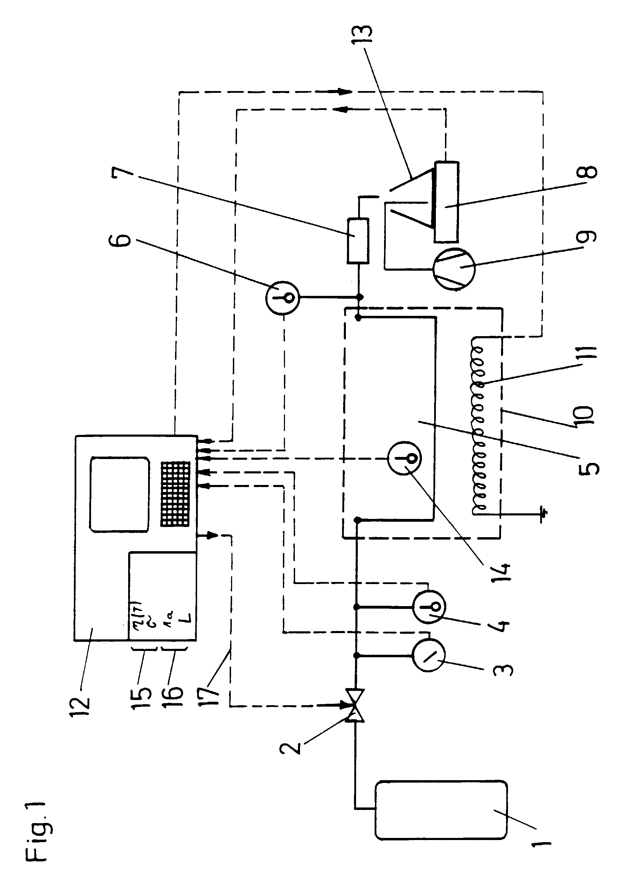

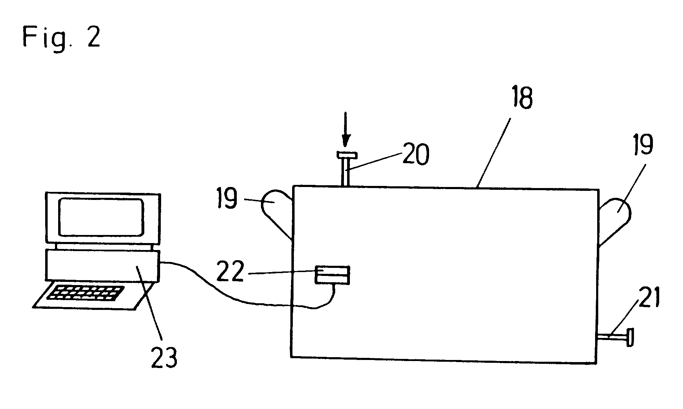

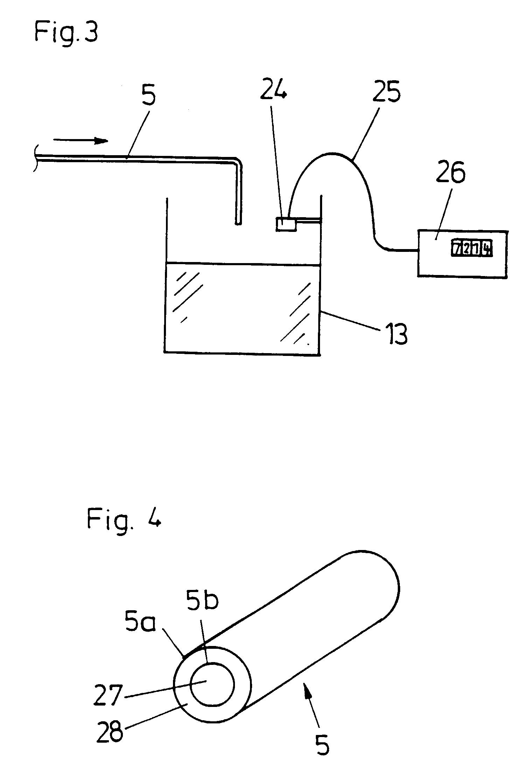

Die Fig. 1 zeigt schematisch ein erstes Ausführungsbeispiel, Fig. 2 zeigt eine tragbare Variante, Fig. 3 zeigt eine Variante mit Füllstandsmessung und Fig. 4 zeigt eine als Doppelrohr ausgebildete Röhre.

Die im Ausführungsbeispiel dargestellte Vorrichtung weist folgende Elemente auf:

- ein Druckreservoir 1 der zu testenden Flüssigkeit (Leitung, Blasenspeicher, Reaktionstank unter Druck)
- eine Röhre/Schlauch 5 aus dem Material (Stahl, Kupfer, Glas, Kunststoffe ...) auf das abgelagert werden soll; geeignet sind auch mit dem Material innenbeschichtete Rohre/Schläuche
- ein Regelventil 2
- einen Drucksensor 3 am Eingang in die Röhre/Schlauch 5
- eine Temperaturmessung 4 am Eingang in die Röhre/Schlauch 5
- eine Temperaturmessung 6 am Ausgang der Röhre/Schlauch 5
- ein Wärmebad 10 (oder einen Heizblock oder eine Heißluftströmung) mit entsprechend genauer Temperaturregelung 11, 14

- eine hochgenaue Massendurchflußmessung in Form einer Waage 8
- eine Datenerfassungsstation und Auswertvorrichtung 12, die vorzugsweise einen Regler enthält, der in der Lage ist, Parameter, die sich aus der Verknüpfung verschiedener Meßgrößen ergeben, zu regeln (über das Regelventil 2)
- einen Kühler 7 am Ausgang der Röhre 5 bzw. des Schlauches, um Verdampfungsverluste zu vermeiden
- gegebenenfalls eine Vorrichtung und Steuerung zum automatischen Reinigen (Säure, Lauge) und Spülen (reines Lösungsmittel) der Röhre/Schlauch
- gegebenenfalls eine Pumpe 9 zum Entleeren des Auffangbehälters 13 nach einer Messung/Reinigung/ Spülung der Röhre/Schlauch.

Die Druckdifferenz zwischen Eingang und Ausgang der Röhre 5 wird durch einen Drucksensor 3 am Eingang der Röhre erfaßt, wobei am Ausgang der Röhre Atmosphärendruck herrscht. Dies erlaubt es, mit einem Drucksensor auszukommen. Selbstverständlich ist es auch möglich, auch am Ausgang einen Drucksensor anzuordnen und dann die Druckdifferenz exakt zu messen.

Für die Erfindung ist es wichtig, die Durchflußrate genau zu kennen. Mit bisherigen Kolbenpumpen gab es dabei Schwierigkeiten. Vor allem war mit hochgenauen Dosierpumpen nur ein intermittierender Betrieb und kein stetiger Durchfluß möglich. Zur Erzielung eines stetigen Durchflusses ist gemäß einer Variante der Erfindung ein Druck- und Flüssigkeitsreservoir am Eingang vorgesehen, wobei zwischen diesem und der Röhre 5 ein Regelventil 2 angeordnet ist. Das Druck- und Flüssigkeitsreservoir 1 erlaubt es Flüssigkeit kontinuierlich zu fördern, wobei das Regelventil nach gewünschten Vorgaben den Durchfluß steuert.

Zur hochpräzisen Erfassung der Durchflußrate ist gemäß einer Variante der Erfindung vorgesehen, das Gewicht der durchgetretenen Flüssigkeit bzw. Schmelze zu erfassen. Dies kann mittels einer Wägeeinrichtung 8 geschehen, die einen Behälter 13 wiegt, in den die Flüssigkeit strömt. Grundsätzlich wäre es auch möglich, das Druck- und Flüssigkeitsreservoir 1 am Anfang zu messen und dessen Gewichtsabnahme zu erfassen.

Das Wasserbad 10 beheizt die Röhre 5 von außen. Die Wassertemperatur wird über einen Sensor 14 erfaßt und über einen Temperaturregler in der Auswertvorrichtung 12 über die Heizmittel 11 auf einen konstanten Wert geregelt.

Die elektronische Auswertvorrichtung 12 weist Eingänge auf, die Signale von der Erfassungseinrichtung 8 für die Durchflußrate, von der Erfassungseinrichtung 3 für die Druckdifferenz, von der Erfassungseinrichtung 14 für die Außentemperatur der Röhre 5 und schließlich von den beiden erfindungsgemäßen Temperatursensoren 4, 6 vor und nach der Röhre 5 empfangen.

Weiters enthält die Auswertvorrichtung einen Speicher 15, in dem flüssigkeitsspezifische Parameter, beispielsweise die temperaturabhängige Viskosität oder die spezifische Wärmekapazität gespeichert sind. Auch einrichtungsspezifische Parameter, wie beispielsweise die Länge der Röhre 5 und deren Außendurchmesser, können im Speicher 16 der Auswertvorrichtung 12 abgespeichert werden. Eine programmgesteuerte Recheneinheit kann nun nach den oben angeführten Formeln aus den von den Erfassungseinrichtungen 3, 4, 6, 8, 14 erfaßten Daten und den in den Speichern 15, 16 gespeicherten Parametern die gewünschte Abscheiderate über den Innenradius der Röhre bzw. des Schlauches ermitteln. In der Praxis hat sich gezeigt, daß Innendurchmesser von etwa 0,2 bis etwa 2 mm günstig sind, um die Anlagerung von Kalk zu studieren.

Die Auswertvorrichtung 12 kann über eine Leitung 17 das Regelventil 2 ansteuern, wobei es möglich ist, eine der Meßgrößen, beispielsweise die Druckdifferenz oder die Durchflußrate während des gesamten Meßvorganges konstant zu halten. Dies erlaubt es, reproduzierbare Meßvorgänge durchzuführen. Grundsätzlich ist es aber nicht nötig, eine dieser Meßgrößen konstant zu halten. Vielmehr reicht es aus, diese Meßgrößen zu jedem Zeitpunkt genau zu kennen.

Die erfindungsgemäße Einrichtung eignet sich insbesondere zur Überprüfung von physikalischen (beispielsweise elektrostatischen oder magnetischen Wasserbehandlungsgeräten). Mit der erfindungsgemäßen Einrichtung kann überprüft werden, ob sich das Anlagern von Kalk im Inneren der Röhre bzw. des Schlauches durch Vorschalten eines physikalischen Wasserbehandlungsgerätes verringert. Dabei liegt ein besonderer Vorteil der Erfindung darin, daß eine genaue quantitative Erfassung möglich ist.

Die oben beschriebene Einrichtung eignet sich auch zur Durchführung des erfindungsgemäßen Verfahrens, mit dem es möglich ist, die Abscheideraten nicht nur qualitativ, sondern auch quantitativ zu erfassen. Erfindungsgemäß wird dazu die Schmelze durch einen Schlauch oder eine Röhre geleitet und quantitativ erfaßt, wie sich der Innenradius durch Abscheiden verringert.

Um den Radius erfassen zu können, kann die HagenPoiseuille[sche] Beziehung (obige Formel 2) ausgenutzt werden. Man muß dazu die Druckdifferenz, die Durchflußrate und die Viskosität kennen. Die Messung der Durchflußrate und der Druckdifferenz bereiten keine Probleme. Die Viskosität ist allerdings im allgemeinen stark temperaturabhängig. In einem solchen Fall, erfaßt man daher günstigerweise die mittlere Temperatur innerhalb der Röhre bzw. des Schlauches. Bei engen Röhren und Schläuchen ist die direkte Temperaturerfassung unter Umständen schwierig. Man kann aber auf die oben beispielsweise anhand der Formeln 3 bis 5 dargestellten Vorgangsweise durch einfache Messung der Außentemperatur und der Temperaturen am Eingang und Ausgang auf die mittlere Temperatur in der Röhre kommen.

Anstelle der Wägung zur Erfassung der Durchflußrate ist auch eine Füllstandsmessung in einem Auffanggefäß möglich, z.B. über einen Schwimmer oder einen Ultra-schallsensor.

Die Fig. 2 zeigt eine tragbare Variante. In einem tragbaren Transportgehäuse 18 mit Traggriffen 19 sind die wesentlichen Komponenten der erfindungsgemäßen Einrichtung untergebracht. Ein Wasseranschluß 20 erlaubt die Zufuhr des zu überprüfenden Wassers. Ein Auslaß 21 dient zum Ablassen des geprüften Wassers. Die Elektronik zur Auswertung kann entweder in der Baueinheit 18 oder extern angeordnet sein. Im letzteren Fall ist eine Schnittstelle 22 zum Anschluß eines Computers 23 vorgesehen.

Die Fig. 3 zeigt eine alternative Methode bzw. Vorrichtung zur Ermittlung der Durchflußmenge. Es wird dazu der Füllstand im Auffanggefäß 13 ermittelt, beispielsweise über einen Ultraschallsensor 24, der die Entfernung zur Flüssigkeitsoberfläche mißt. Der Ultraschallsensor 24 ist über ein Kabel 25 mit einer Auswerteinrichtung 26 verbunden.

Im allgemeinen wird man die zu untersuchende Flüssigkeit der Einfachheit halber durch eine Röhre bzw. einen Schlauch (mit kapillaren Abmessungen) führen. Grundsätzlich ist es aber auch möglich, die Flüssigkeit anders mit definierten Strömungsbedingungen zu führen, insbesondere in Spaltströmungen (beispielsweise kreisringförmiger Spalt oder rechteckiger bzw. quadratischer Spalt).

In Fig. 4 ist ein Doppelrohr 5 mit einem Außenrohr 5a und einem Innenrohr 5b gezeigt. Die zu untersuchende Flüssigkeit kann beispielsweise im Kreisringspalt 28 strömen. Der innere Bereich 27 kann entweder ungenutzt sein oder aber von einem Heizmedium durchströmt sein. Eine solche Anordnung entspricht dem klassischen Röhrenwärmeaustauscher. Fließen das heizende Medium im Innenkanal 27 und das beheizende Medium im Außenkanal 28 in die gleiche Richtung, liegt ein Gleichstromwärmeaustauscher vor.

**Patentansprüche**

1. Einrichtung zur Bestimmung von Abscheideraten von gelösten Inhaltsstoffen einer Flüssigkeit oder Schmelze an einer Oberfläche

   - mit einer beheizten Röhre oder einem Schlauch, durch welche(n) die Flüssigkeit oder Schmelze strömt,
   - mit einer Einrichtung zur Erfassung der Durchflußrate durch die Röhre bzw. Schlauch,
   - mit einer Einrichtung zur Erfassung der Druckdifferenz zwischen Eingang und Ausgang der Röhre bzw. des Schlauches,
   - mit einer Einrichtung zur Erfassung der Außentemperatur der Röhre bzw. des Schlauches,
   - mit einer Einrichtung (4, 6) zur Erfassung der Temperatur der Flüssigkeit bzw. der Schmelze sowohl vor als auch nach der Röhre (5) bzw. dem Schlauch, wobei eine elektronische Auswertvorrichtung (12) vorgesehen ist, deren Eingänge mit der Erfassungseinrichtung (8) der Durchflußrate, mit der Erfassungseinrichtung (3) der Druckdifferenz, mit der Erfassungseinrichtung (14) der Außentemperatur der Röhre bzw. Schlauches, und mit der Erfassungseinrichtung (4, 6) der Temperatur der Flüssigkeit bzw. der Schmelze sowohl vor als auch nach der Röhre bzw. dem Schlauch verbunden sind, wobei die Auswerteinrichtung (12) weiters einen Speicher (15, 16), in dem flüssigkeitsspezifische, z.B. Viskosität $\eta(T)$, und einrichtungsspezifische Parameter, z.B. Länge L der Röhre oder des Schlauches und Außenradius $r_a$ der Röhre oder des Schlauches, abgespeichert werden, und eine programmgesteuerte Recheneinheit zum quantitativen Berechnen der Abscheiderate aus allen von den genannten Erfassungseinrichtungen erfaßten Daten und allen genannten gespeicherten Parametern aufweist.

2. Einrichtung nach Anspruch 1, dadurch gekennzeichnet, daß die Röhre (5) bzw. der Schlauch über ein Wasserbad (10) von außen beheizt ist, dessen Wassertemperatur erfaßt wird.

3. Einrichtung nach Anspruch 1, dadurch gekennzeichnet, daß die Röhre (5) bzw. der Schlauch über einen umgebenden Wärmeblock beheizt ist, dessen Temperatur erfaßt wird.

4. Einrichtung nach einem der Anspruche 1 bis 3, dadurch gekennzeichnet, daß der Röhre (5) oder dem Schlauch ein Kühler (7) nachgeschaltet ist.

5. Einrichtung nach einer der Anspruche 1 bis 4, dadurch gekennzeichnet, daß die Röhre oder der Schlauch kreisringförmigen Querschnitt aufweist, wobei der Innendurchmesser 0,2 bis 2 mm beträgt.

6. Einrichtung nach einem der Ansprüche 1 bis 4, dadurch gekennzeichnet, daß die Röhre oder der Schlauch einen beispielsweise kreisringförmigen Strömungsspalt aufweist.

7. Einrichtung nach einem der Ansprüche 1 bis 6, dadurch gekennzeichnet, daß die Flüssigkeit oder Schmelze über einen Wärmetauscher, vorzugsweise einen Röhrenwärmetauscher beheizt ist.

8. Einrichtung nach einem der Ansprüche 1 bis 7, dadurch gekennzeichnet, daß die Röhre oder der Schlauch einen zwei- oder mehrwandigen Aufbau mit zumindest zwei Strömungskanälen aufweist, wobei in einem Strömungskanal die zu untersuchende Flüssigkeit oder Schmelze strömt und im anderen Strömungskanal ein Heizfluid strömt.

9. Einrichtung nach einem der Ansprüche 1 bis 6, dadurch gekennzeichnet, daß die Einrichtung zur Erfassung der Druckdifferenz zwischen Eingang und Ausgang der Röhre (5) bzw. des Schlauches einen Drucksensor (3) im Bereich des Eingangs aufweist und der Druck am Ausgang der Atmosphärendruck ist.

10. Einrichtung nach einem der Ansprüche 1 bis 9, dadurch gekennzeichnet, daß zur Ermittlung der Durchflußrate durch die Röhre (5) bzw. den Schlauch das Gewicht der durchtretenden Flüssigkeit bzw. Schmelze erfaßt wird.

11. Einrichtung nach Anspruch 10, dadurch gekennzeichnet, daß eine Wägeeinrichtung (8) vorgesehen ist, die einen Behälter (13) wiegt, in den bzw. aus dem die Flüssigkeit bzw. Schmelze strömt.

12. Einrichtung nach einem der Ansprüche 1 bis 9, dadurch gekennzeichnet, daß zur Ermittlung der Durchflußrate durch die Röhre (5) bzw. den Schlauch der Füllstand in einem Auffanggefäß für die durchgetretene Flüssigkeit bzw. Schmelze erfaßt wird.

13. Einrichtung nach einem der Ansprüche 1 bis 12, dadurch gekennzeichnet, daß ein Druck- und Flüssigkeitsreservoir (1) vorgesehen ist und zwischen diesem und der Röhre (5) bzw. dem Schlauch ein Ventil, vorzugsweise ein Regelventil, angeordnet ist.

14. Einrichtung nach einem der Ansprüche 1 bis 13, dadurch gekennzeichnet, daß die Auswertvorrichtung (12) das Regelventil (2) ansteuert, wobei eine der Meßgrößen, beispielsweise die Druckdifferenz oder die Durchflußrate während des gesamten Meßvorganges konstant gehalten ist.

15. Einrichtung nach einem der Anspruche 1 bis 14, dadurch gekennzeichnet, daß die Komponenten der Einrichtung zu einer kompakten tragbaren Baueinheit (18) zusammengefaßt sind.

16. Verwendung einer Einrichtung nach einem der Ansprüche 1 bis 15 zur Überprüfung eines physikalischen Wasserbehandlungsgerätes, wobei von diesem behandeltes Wasser durch die Röhre bzw. Schlauch der Einrichtung fließt.

17. Verfahren zur quantitativen Bestimmung von Abscheideraten von gelösten Inhaltsstoffen einer Flüssigkeit oder Schmelze an einer Oberfläche, wobei die Flüssigkeit oder Schmelze durch einen Schlauch oder eine Röhre geleitet wird und quantitativ erfaßt wird, wie sich der Innenradius in der Röhre bzw. im Schlauch durch das Abscheiden von Inhaltsstoffen an der inneren Oberfläche verringert, wobei die Druckdifferenz über dem Schlauch bzw. der Röhre, die Durchflußrate durch den Schlauch bzw. die Röhre und die Viskosität der Flüssigkeit bzw. Schmelze im Schlauch bzw. der Röhre ermittelt wird und daraus aus der Hagen-Poiseuille[schen] Formel der Innenradius berechnet wird.

18. Verfahren nach Anspruch 17, dadurch gekennzeichnet, daß der Schlauch bzw. die Röhre beheizt ist.

19. Verfahren nach einem der Ansprüche 17 bis 18, dadurch gekennzeichnet, daß die mittlere Temperatur in der Röhre bzw. im Schlauch erfaßt wird und der temperaturabhängige Viskositätswert bei dieser mittleren Temperatur genommen wird.

20. Verfahren nach Anspruch 19, dadurch gekennzeichnet, daß die mittlere Temperatur $T_m$ durch Messung der Außentemperatur TO der Röhre bzw. des Schlauches sowie durch Messung der Temperatur (T_ein, T_aus) der Flüssigkeit oder Schmelze am Eingang und Ausgang der Röhre oder des Schlauches ermittelt wird, wobei die mittlere Temperatur gegeben ist durch

$$T_m = TO + \frac{T\_ein\text{-}TO}{\tau}[1\text{-}\exp(-\tau.L)]$$

mit $\tau = 2 \pi k\, r_a/W$, wobei L die Rohrlänge, $r_a$ der Außendurchmesser, k der Wärmedurchgangskoeffizient und W der Wasserwert sind.

**21.** Verfahren nach Anspruch 19, dadurch gekennzeichnet, daß als Viskositätswert die mittlere Viskosität nach der folgenden Formel

$$\langle \eta \rangle = \frac{\int_0^L \eta(z)dz}{L} = \frac{\int_{T_{ein}}^{T_{aus}} \eta(z(T)) \cdot z'(T) \cdot dT}{T_{aus} - T_{ein}} \qquad (6)$$

genommen wird.

## Claims

**1.** Apparatus for determining deposit rates of dissolved substances in a liquid or molten material at a surface, comprising

- a heated tube or a hose through which the liquid or molten material flows,
- a device for detecting the flow rate through the tube or hose,
- a device for detecting the pressure difference between the inlet and the outlet of the tube or hose,
- a device for detecting the outside temperature of the tube or hose, and
- a device (4, 6) for detecting the temperature of the liquid or the molten material both upstream and also downstream of the tube (5) or the hose,

   wherein there is provided an electronic evaluation apparatus (12) whose inputs are connected to the device (8) for detecting the flow rate, the device (3) for detecting the pressure difference, the device (14) for detecting the outside temperature of the tube or hose, and the device (4, 6) for detecting the temperature of the liquid or the molten material both upstream and also downstream of the tube or hose, wherein the evaluation apparatus (12) further has a memory (15, 16) in which liquid-specific parameters, for example viscosity $\eta(T)$, and apparatus-specific parameters, for example length L of the tube or hose and outside radius $r_a$ of the tube or hose are stored, and a program-controlled computing unit for quantitative calculation of the deposit rate from all data detected by said detection devices and all said stored parameters.

**2.** Apparatus according to claim 1 characterised in that the tube (5) or the hose is heated from the exterior by way of a water bath (10) whose water temperature is detected.

**3.** Apparatus according to claim 1 characterised in that the tube (5) or the hose is heated by way of a surrounding heating block whose temperature is detected.

**4.** Apparatus according to one of claims 1 to 3 characterised in that a cooler (7) is connected downstream of the tube (5) or the hose.

**5.** Apparatus according to one of claims 1 to 4 characterised in that the tube or the hose is of an annular cross-section, the inside diameter being between 0.2 and 2 mm.

**6.** Apparatus according to one of claims 1 to 4 characterised in that the tube or the hose has a for example annular flow gap.

**7.** Apparatus according to one of claims 1 to 6 characterised in that the liquid or molten material is heated by way of a heat exchanger, preferably a tube-type heat exchanger.

**8.** Apparatus according to one of claims 1 to 7 characterised in that the tube or the hose is of a two- or multi-wall structure with at least two flow passages, wherein the liquid or molten material to be investigated flows in one flow passage and a heating fluid flows in the other flow passage.

9.  Apparatus according to one of claims 1 to 6 characterised in that the device for detecting the pressure difference between the inlet and the outlet of the tube (5) or the hose has a pressure sensor (3) in the region of the inlet and the pressure at the outlet is atmospheric pressure.

10. Apparatus according to one of claims 1 to 9 characterised in that the weight of the liquid or molten material passing through the tube (5) or the hose is detected to ascertain the flow rate through the tube (5) or the hose.

11. Apparatus according to claim 10 characterised in that there is provided a weighing device (8) which weighs a container (13) into which or out of which the liquid or molten material flows.

12. Apparatus according to one of claims 1 to 9 characterised in that the filling level in a catch vessel for the liquid or molten material which has passed through the tube or hose is detected for ascertaining the flow rate through the tube (5) or the hose.

13. Apparatus according to one of claims 1 to 12 characterised in that there is provided a pressure and liquid reservoir (1) and arranged between the reservoir and the tube (5) or the hose is a valve, preferably a regulating valve.

14. Apparatus according to one of claims 1 to 13 characterised in that the evaluation apparatus (12) actuates the regulating valve (2), wherein one of the measuring values, for example the pressure difference or the flow rate, is kept constant during the entire measuring operation.

15. Apparatus according to one of claims 1 to 14 characterised in that the components of the apparatus are combined together to form a compact portable unit (18).

16. Use of an apparatus according to one of claims 1 to 15 for checking a physical water treatment installation, wherein water treated thereby flows through the tube or hose of the apparatus.

17. A method of quantitatively determining deposit rates of dissolved substances in a liquid or molten material at a surface, wherein the liquid or molten material is passed through a hose or a tube and the way in which the inside radius in the tube or hose is reduced by the deposit of substances contained in the liquid or molten material at the inside surface is quantitatively detected, wherein the pressure difference across the hose or tube, the flow rate through the hose or tube and the viscosity of the liquid or molten material in the hose or tube is ascertained and the inside radius is ascertained therefrom from Hagen-Poiseuille's formula.

18. A method according to claim 17 characterised in that the hose or tube is heated.

19. A method according to one of claims 17 and 18 characterised in that the mean temperature in the tube or hose is detected and the temperature-dependent viscosity value is taken at that mean temperature.

20. A method according to claim 19 characterised in that the mean temperature $T_m$ is ascertained by measurement of the outside temperature T0 of the tube or hose and by measurement of the temperature (T_ein, T_aus) of the liquid or molten material at the inlet and outlet of the tube or hose, wherein the mean temperature is given by:

$$T_m = TO + \frac{T\_ein\text{-}TO}{\tau}[1\text{-}\exp(\text{-}\tau.L)]$$

with $= 2\,k\,r_a/W$, wherein L is the tube length, $r_a$ is the outside diameter, k is the heat transfer coefficient and W is the water value.

21. A method according to claim 19 characterised in that the mean viscosity in accordance with the following formula:

$$\langle\eta\rangle = \frac{\int\limits_{0}^{L}\eta(z)dz}{L} = \frac{\int\limits_{T_{ein}}^{T_{aus}}\eta(z(T))\cdot z'(T)\cdot dT}{T_{aus} - T_{ein}} \qquad (6)$$

is taken as the viscosity value.

## Revendications

1. Dispositif pour la détermination des vitesses de dépôt de constituants dissouts d'un liquide ou d'une fonte au niveau d'une surface,

   - avec un tube chauffé ou avec un tuyau flexible à travers lequel s'écoule le liquide ou la fonte,
   - avec un dispositif d'enregistrement du débit à travers le tube ou le tuyau flexible,
   - avec un dispositif d'enregistrement de la différence de pression entre l'entrée et la sortie du tube ou du tuyau flexible,
   - avec un dispositif d'enregistrement de la température extérieure du tube ou du tuyau flexible,
   - avec un dispositif (4, 6) d'enregistrement de la température du liquide ou de la fonte, aussi bien avant qu'après le tube (5) ou le tuyau flexible, un dispositif d'exploitation électronique (12) étant prévu, dont les entrées sont reliées au dispositif d'enregistrement du débit (8), au dispositif d'enregistrement de la différence de pression (3), au dispositif d'enregistrement de la température extérieure du tube ou du tuyau flexible (14) et au dispositif d'enregistrement (4, 6) de la température du liquide ou de la fonte, aussi bien avant qu'après le tube ou le tuyau flexible, le dispositif d'exploitation (12) présentant en outre une mémoire (15, 16), dans laquelle les paramètres spécifiques, par exemple la $\eta(T)$ du liquide et un paramètre spécifique de construction du dispositif, par exemple la longueur L du tube ou du tuyau flexible, ainsi que le rayon extérieur $r_a$ du tube ou du tuyau flexible sont mémorisés, une unité de calcul à commande programmable pour le calcul quantitatif de la vitesse de dépôt à partir de l'ensemble des données enregistrées par les dispositifs d'enregistrement indiqués et de l'ensemble des paramètres stockés en mémoire indiqués.

2. Dispositif selon la revendication 1, caractérisé en ce que le tube (5) ou le tuyau flexible est chauffé de l'extérieur par l'intermédiaire d'un bain d'eau, dont la température est enregistrée.

3. Dispositif selon la revendication 1, caractérisé en ce que le tube (5) ou le tuyau flexible est chauffé par l'intermédiaire d'un bloc chauffant l'entourant, dont la température est enregistrée.

4. Dispositif selon l'une des revendications 1 à 3, caractérisé en ce qu'en aval du tube (5) ou du tuyau flexible est disposé un réfrigérant (7).

5. Dispositif selon l'une des revendications 1 à 4, caractérisé en ce que le tube ou le tuyau flexible présente une section circulaire, le diamètre interne mesurant de 0,2 à 2 mm.

6. Dispositif selon l'une des revendications 1 à 4, caractérisé en ce que le tube ou le tuyau flexible présente une fente de passage par exemple circulaire.

7. Dispositif selon l'une des revendications 1 à 6, caractérisé en ce que le liquide ou la fonte sont chauffés par l'intermédiaire d'un échangeur de chaleur, de préférence un échangeur de chaleur tubulaire.

8. Dispositif selon l'une des revendications 1 à 7, caractérisé en ce que le tube ou le tuyau flexible présente une construction à deux parois ou plus, avec au moins deux canaux d'écoulement, le liquide ou la fonte à examiner circulant dans un canal d'écoulement et un fluide de chauffage dans l'autre canal.

9. Dispositif selon l'une des revendications 1 à 6, caractérisé en ce que le dispositif d'enregistrement de la différence de pression entre l'entrée et la sortie du tube (5) ou du tuyau flexible comporte un capteur de pression (3) dans la zone d'entrée, et en ce que la pression en sortie est la pression atmosphérique.

10. Dispositif selon l'une des revendications 1 à 9, caractérisé en ce que, pour la détermination du débit à travers le tube (5) ou le tuyau flexible, on enregistre le poids du liquide ou de la fonte passant à travers.

11. Dispositif selon la revendication 10, caractérisé en ce qu'un dispositif de pesée (8) est prévu, qui pèse un récipient (13), dans lequel ou à partir duquel s'écoule le liquide ou la fonte.

12. Dispositif selon l'une des revendications 1 à 9, caractérisé en ce que, pour la détermination du débit à travers le tube (5) ou le tuyau flexible, on enregistre le niveau de remplissage d'un récipient de réception destiné au liquide ou à la fonte écoulé.

13. Dispositif selon l'une des revendications 1 à 12, caractérisé en ce qu'un réservoir de pression et de liquide (1) est prévu et qu'entre celui-ci et le tube (5) ou le tuyau flexible, une valve, de préférence, une valve de régulation, est prévue.

14. Dispositif selon l'une des revendicaticns 1 à 13, caractérisé en ce que le dispositif d'exploitation (12) commande la valve de régulation (2), une grandeur de mesure, par exemple la différence de pression ou le débit, étant maintenue constante pendant la totalité ou processus de mesure.

15. Dispositif selon l'une des revendications 1 à 14, caractérisé en ce que les composants du dispositif sont rassemblés pour former un module portable compact (18).

16. Utilisation d'un dispositif selon l'une des revendications 1 à 15 pour contrôler un appareil de traitement de l'eau physique, l'eau traitée par cet appareil s'écoulant à travers le tube ou le tuyau flexible du dispositif.

17. Procédé de détermination quantitative des vitesses de dépôt de constituants dissouts d'un liquide ou d'une fonte au niveau d'une surface, dans lequel le liquide ou la fonte s'écoule à travers un tuyau flexible ou un tube et la marge dans laquelle le diamètre interne du tube ou du tuyau flexible diminue par dépôt de constituants sur les surfaces internes est enregistrée quantitativement, la différence de pression à travers le tube ou le tuyau flexible, le débit à travers le tuyau flexible ou le tube, et la viscosité du liquide ou de la fonte dans le tuyau flexible ou le tube étant déterminés et, à partir de là, le diamètre interne étant calculé à l'aide de la formule de Hagen-Poiseuille.

18. Procédé selon la revendication 17, caractérisé en ce que le tuyau flexible ou le tube est chauffé.

19. Procédé selon l'une des revendications 17 à 18, caractérisé en ce que la température moyenne dans le tube ou le tuyau flexible est enregistrée et que la valeur de la viscosité, dépendant de la température, est prise à cette température moyenne.

20. Procédé selon la revendication 19, caractérisé en ce que la température moyenne $T_m$ est déterminée par mesure de la température extérieure TO du tube ou du tuyau flexible et par mesure de la température (Te, Ts) du liquide ou de la fonte à l'entrée et à la sortie du tube ou du tuyau flexible, la température moyenne étant donnée par

$$Tm = TO + (Te - TO)[1 - \exp(-\tau.L)]/\tau$$

avec $\tau = 2 \pi k r_a /W$, L étant la longueur du tube, $r_a$ son rayon extérieur, k le coefficient de transfert thermique et W l'équivalent en eau.

21. Procédé selon la revendication 19, caractérisé en ce que comme valeur de la viscosité, on prend la viscosité moyenne d'après la formule suivante :

$$\langle \eta \rangle = \frac{\displaystyle\int_0^L \eta(z)\,dz}{L} = \frac{\displaystyle\int_{T_e}^{T_s} \eta(z(T)) \cdot z'(T) \cdot dT}{T_s - T_e}$$

Fig.1

EP 0 754 299 B1

Fig. 2

EP 0 754 299 B1

Fig. 3

Fig. 4